(19) Europäisches Patentamt
European Patent Office
Office européen des brevets

(11) Numéro de publication: 0 027 759
B1

(12) FASCICULE DE BREVET EUROPEEN

(45) Date de publication du fascicule du brevet: 12.10.83

(51) Int. Cl.³: C 07 D 307/94,
A 61 K 31/34 //C07C91/23

(21) Numéro de dépôt: 80401437.1

(22) Date de dépôt: 08.10.80

# (54) Nouveaux dérivés spiranniques, procédé pour leur préparation, et médicaments les contenant.

(30) Priorité: 09.10.79 FR 7925041

(43) Date de publication de la demande:
29.04.81 Bulletin 81/17

(45) Mention de la délivrance du brevet:
12.10.83 Bulletin 83/41

(84) Etats contractants désignés:
AT BE CH DE FR GB IT LI LU NL SE

(56) Documents cités:
JOURNAL OF THE AMERICAN CHEMICAL SOCIETY, vol. 101, no. 2, 17 janvier 1979 WASHINGTON, D.C. (US) D. A. EVANS et al.: "Phosphonamide Stabilized Allylic Carbanions. New Homoenolate Anion Equivalents" pages 371—378
CHEMICAL ABSTRACTS, vol. 84, no. 9 1er mars 1976, pages 493, 494, réf. 59167x COLUMBUS, OHIO (US)

(73) Titulaire: ETABLISSEMENTS NATIVELLE S.A.
27, rue de la Procession
F-75015 Paris (FR)

(72) Inventeur: Tsatsas, Georges
121 avenue de la Reine Sophie
Athènes (GR)
Inventeur: Costakis, Evan E.
6 rue Zefiron
Agia Paraskevi, Attikis (GR)
Inventeur: Foscolos, Georges V.
1, rue Mitroou
Le Pirée (GR)

(74) Mandataire: L'Helgoualch, Jean et al,
OFFICE PICARD 134 Boulevard de Clichy
F-75018 Paris (FR)

EP 0 027 759 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveaux dérivés spiranniques, procédé pour leur préparation, et médicaments les contenant

La présente invention concerne des dérivés spiranniques, et plus particulièrement des aryl-4-aminoalkyl-3-oxa-1 spiroalcanes, et des aryl-4 aminoalkyl-3 oxa-1 spiroalcanones-2, un procédé pour leur préparation, et leur application en thérapeutique.

Des aminométhyl-3 oxo-2 oxa-1 spiro(4,5)-decanes ont été décrits dans Chemical Abstracts, vol. 84, n°9 (1.3.76) 59167x.

Les dérivés spiranniques conformes à la présente invention peuvent être représentés par la formule générale (I):

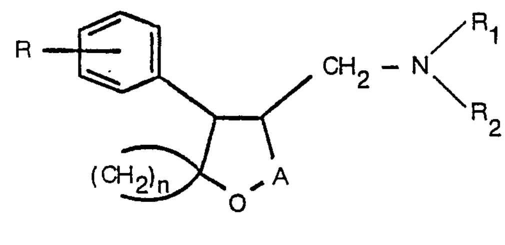

dans laquelle R est un atome d'hydrogène ou d'halogène, ou un groupe méthyle; $R_1$ et $R_2$ identiques ou différents, représentent un atome d'hydrogène ou un groupe méthyle, ou représentent ensemble une chaîne carbonée formant avec l'atome d'azote auquel ils sont rattachés, un hétérocycle à 5 ou 6 chaînons; A représente un groupe

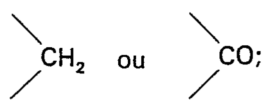

et n est 4 ou 5.

Quand $R_1$ et $R_2$ forment un hétérocycle azoté avec l'atome d'azote auquel ils sont rattachés, cet hétérocycle comportant 5 ou 6 chaînons peut être par exemple un groupe pyrrolyle, pyrrolidinyle, pipéridinyle.

L'invention a également pour objet un procédé de préparation des composés de formule générale I à partir des $\beta$-hydroxyacides de formule générale (II):

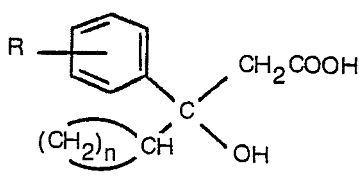

(II)

dans laquelle R et n ont la même signification que dans la formule (I), par action d'un acide fort usuel tel que l'acide sulfurique concentré, suivie d'une hydrolyse. La réaction s'effectue par transposition intramoléculaire suivant le principe décrit par G. Tsatas et G. Cottakis dans "Bull.Soc.Chim. de France (1970) page 3609, et on obtient ainsi une $\gamma$-spirolactone de formule générale (III)

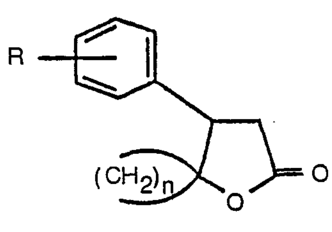

(III)

dans laquelle R et n conservent la définition indiquée dans la formule générale (I).

Par action du carbonate de méthyle et de magnésium sur la spirolactone de formule générale (III), on obtient l'$\alpha$-carboxy-$\gamma$-spirolactone correspondante, de formule générale (IV):

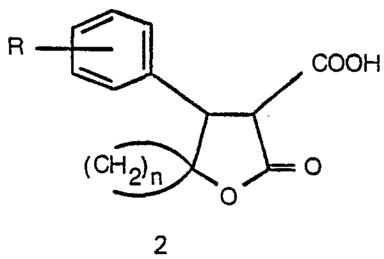

que l'on transforme en composé de formule générale (I) suivant l'invention, dans laquelle A représente un groupe

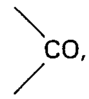

par action d'une amine aliphatique appropriée, en présence de formol ou de paraformaldéhyde suivant la réaction de Mannich.

L'acide fort peut être choisi parmi les acides forts usuels de la technique, et par exemple, l'acide sulfurique concentré, de préférence en présence d'un oléum, l'acide chlorhydrique, l'acide p-toluène sulfonique, un super acide ou un acide de Lewis.

Suivant une variante du procédé conforme à la présente invention, les composés de formule générale (I) dans laquelle A est

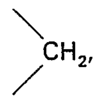

sont obtenus à partir des composés de formule générale I où A est

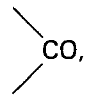

par ouverture réductive au moyen de l'hydrure de lithium aluminium pour former un diol, puis cyclisation par déshydratation interne par action du chlorure de p-bromobenzène sulfonyle dans la pyridine.

Les $\beta$-hydroxyacides de formule générale (II) utilisés comme composés de départ pour la préparation des dérivés spiranniques de formule générale (I) conformes à l'invention, peuvent être aisément obtenus par des procédés connus, et par exemple en faisant réagir un ester halogéné tel que le bromoacétate d'éthyle, du zinc, et une cycloalkylarylcétone appropriée, suivant la réaction de Reformatsky, suivie d'une saponification de l'ester obtenu.

L'invention s'étend également aux sels des dérivés spiranniques de formule générale (I) et en particulier aux sels pharmaceutiquement acceptables, par réaction avec les acides usuels tels que les acides chlorhydrique, sulfurique, phosphorique, acétique, propionique, oxalique, lactique, citrique, tartrique, ou malonique. On peut également préparer des sels en faisant réagir un halogénure d'alkyle sur les composés spiranniques conformes à l'invention. Les sels peuvent être obtenus de façon usuelle enfaisant réagir en proportions sensiblement stoechiométriques un dérivé spirannique conforme à l'invention avec un acide approprié, dans un solvant convenablement choisi, tel qu'un alcool, une cétone, un solvant chloré, ou un éther.

L'invention a également pour objet l'application des dérivés spiranniques de formule générale I et de leurs sels pharmaceutiquement acceptables, à titre de principe actif de médicament. Les expérimentations pharmacologiques et toxicologiques effectuées sur les composés spiranniques de formule générale I ont en effet mis en évidence d'intéressantes propriétés permettant leur application en thérapeutique.

Les expérimentations ont été effectuées sur le rat et la souris en utilisant les dérivés spiranniques conformes à l'invention sous forme de suspension aqueuse dans la gomme arabique, et en les administrant par voie orale sous un volume de 0,1ml par 10g de poids corporel.

La toxicité orale, testée chez la souris, révèle une dose léthale DL 50 de l'ordre de 350 à 400mg/kg.

Les composés spiranniques suivant l'invention sont actifs sur le système nerveux central. Plus particulièrement, ils présentent une activité antiréserpinique et analgésique et procurent des effets proadrénergiques, protryptaminergiques, dopaminergiques et sédatifs, comme indiqué ci-dessous plus en détail en prenant comme exemple spécifique la (diméthylamino-méthyl)-3 phényl-4 oxa-1 spiro (4,5)décane, révélant un profil neuropharmacologique voisin de celui d'un antidépresseur tel que l'imipramine.

Activité antiréserpinique:

Le produit administré après la réserpine s'est révélé capable, à la dose de 25mg/kg, d'antagoniser partiellement l'hypothermie et la ptose palpébrale réserpiniques déjà existantes, démontrant ainsi une activité similaire à celle de 25mg/kg d'imipramine; administré avant la réserpine, il a démontré une certaine activité antagoniste au niveau de l'hypothermie mais cette activité antiréserpinique reste inférieure à celle de l'imipramine.

Effets proadrénergiques et protryptaminergiques:

La potentialisation de la toxicité de l'amphétamine a été recherchée chez la souris. Le produit a manifesté à 25mg/kg une forte activité potentialisatrice de la toxicité alors que l'imipramine est inactive.

Sur le test de la potentialisation de la toxicité de la yohimbine chez la souris, le produit à 5mg/kg s'est révélé plus actif que les antidépresseurs tricycliques (imipramine et amitryptiline).

Effets dopaminergiques:

Sur le test de la catalepsie induit chez le rat par un neuroleptique (la prochlorperazine) le produit a démontré une bonne activité anticataleptique supérieure en durée à celle d'une dose identique d'imipramine (50mg/kg) mais de même que l'imipramine, le molécule n'influence pas les stéréotypies provoquées par la L-dopa chez la souris alors que l'amphétamine et la phenelzine les potentialisent fortement, mais inférieure à celle de la phenelzine.

Effets sédatifs:

La narcose induite chez la souris par le pentobarbital sodique est fortement prolongée après administration de 50mg/kg de produit. Cette activité est supérieure à celle de l'imipramine ou de la phenelzine.

Activité anticonvulsivante:

A l'inverse des antidépresseurs tricycliques, le produit à l'étude s'est révélé pratiquement inactif sur le maximal électrochoc ou sur les convulsions induites par la cardiazole chez le souris.

Activité analgésique:

L'action analgésique a été recherchée:

A/ sur le test de la plaque chauffante qui a mis en évidence pour le produit à 50mg/kg une activité centrale type narcotique comparable à celle de la codéine. Aucun des produits psychotropes déjà cités ne possède semblable activité.

B/ sur la diminution du nombre d'étirements abdominaux douloureux provoqués par l'injection i.p. de phénylbenzoquinone chez la souris. l'activité analgésique du produit est comparable à un analgésique de référence, l'aspirine, et à celle de l'amitryptiline ou de l'amphétamine.

Les composés suivant l'invention présentent également une nette activité anticholinergique aux niveaux central et périphérique vérifiée à des doses de 25mg/kg et 50mg/kg chez la souris.

Les composés spirranniques conformes à l'invention possèdent en outre une activité antiarythmique vérifiée sur l'oreillette isolée de cobaye et sur les arythmies induites par l'aconitine.

Les propriétés pharmacologiques indiquées ci-dessus montrent que les composés spiranniques de l'invention peuvent être appliqués en thérapeutique humaine, notamment pour le traitement des disfonctionnements cérébraux tels que les états dépressifs, dans les domaines d'applications usuelles des neuroleptiques, des antidépresseurs et des stimulants. Ils peuvent également être utilisés pour le traitement des affections cardiaques en raison de leur activité antiarythmique. Les nouveaux composés spiranniques suivant l'invention peuvent être administrés sous les formes usuelles contenant une quantité pharmacologiquement efficace du composé en tant que principe actif, dilué dans des supports pharmaceutiquement utilisables, par exemple sous forme de comprimés, gélules, capsules, dragées, suppositoires, solutés injectables ou sirops.

Les comprimés peuvent par exemple être obtenus par mélange du composé spirannique ou d'un de ses sels, en tant que principe actif, avec des diluants solides tels que le lactose, le mannitol, le sorbitol, l'amidon, la polyvinylpyrrolidone, le stéarate de magnésium ou d'aluminium, la poudre de cellulose, la silice colloidale, le talc.

Les comprimés, de même que les dragées, peuvent être préparés par enrobage afin de former plusieurs couches, suivant une technique classique. Afin de procurer un effetretard on peut utiliser un enrobage d'une ou plusieurs couches de produit usuel tel que la carboxyméthylcellulose, l'acétophtalate de cellulose, ou l'acétate de polyvinyle.

Des solutions injectables peuvent être préparées au moyen de diluants tels que l'eau bidistillée, le propylèneglycol, une solution hydroalcoolique, ou un mélange de ces diluants, de préférence en présence d'un conservateur approprié choisi parmi ceux communément utilisés dans la technique.

Des formes buvables peuvent être également préparées, par exemple des solutés contenant le composé spirannique suivant l'invention dissous dans de l'eau et du glycérol, en présence d'un édulcorant et d'un antioxydant.

Toutes les formulations adaptées aux divers modes d'administration, par voie orale, parentérale, ou rectale, peuvent être utilisées, le médicament étant associé en tant que principe actif aux excipients pharmaceutiquement acceptables convenablement choisis.

La posologie utile peut varier selon le sujet traité, son âge, la gravité de l'affection en cause, et la voie d'administration. A titre indicatif elle peut être de l'ordre de 0,5mg à 20mg par prise, et de 0,5mg à 90mg par jour environ, chez l'homme adulte, pour l'administration orale.

Les exemples suivants illustrent l'invention.

Exemple 1

(Diméthyleaminométhyl)-3 phényl-4 oxa-1 spiro (4,4) nonane-one-2

Dans un mélange de 80ml d'acide sulfurique et de 3ml d'oléum ($H_2S_2O_7$) on ajoute en petites quantités, sous agitation et en refroidissant, 33g d'acide $\beta$-cyclopentyl-$\beta$-hydroxy-hydrocinnamique préparé en chauffant un mélange de cyclopentylphénylcétone, de bromo-acétate d'éthyle et de zinc, et en saponifiant l'ester obtenu par de la potasse. Le complexe formé est agité à 0°C pendant 30 minutes, puis hydrolysé en adjoutant goutte à goutte 300ml d'eau froide.

Le mélange obtenu est extrait à l'éther, les couches éthérées sont lavées à l'eau et au carbonate de sodium à 10%, puis séchées sur sulfate de sodium. Après élimination de l'éther par distillation sous pression réduite, on distille le résidu et on obtient 27,3g (rendement 90%) de phényl-4 oxa-1 spiro (4,4)nonane-one-2, dont la structure est confirmée par l'analyse.

13g de phényl-4 oxa-1 spiro (4,4)nonane-one-2 sont mélangés avec 200ml d'une solution de carbonate de méthyle et de magnésium dans le diméthylformamide ayant une teneur en magnésium de 0,12g par 2,5ml de solution. Le mélange est porté à ébullition pendant 12 h sous atmosphère de dioxyde de carbone, et après refroidissement à la température ambiante il est versé sous vive agitation dans 400 à 500g d'un mélange d'eau et de glace. On ajoute alors lentement, et sous refroidissement, une solution d'acide chlorhydrique à 10% jusqu'à réaction acide, et on laisse reposer pendant la nuit à 0°C.

Le produit solide qui se forme est recueilli par filtration, traité sur bain-marie par une solution à 10% de carbonate de sodium. Le mélange réactionnel est lavé plusieurs fois à l'eau claire afin d'éliminer la spirolactone non carboxylée. Après acidification par une solution à 10% d'acide chlorhydrique, sous agitation et en refroidissant, on laisse reposer pendant la nuit à 0°C. Le produit solide ainsi formé est filtré, lavé à l'eau, et séché sous vide sur $P_2O_5$.

Après recristallisation dans un mélange de benzène anhydre et d'éther de pétrole, on obtient 14,8g (rendement 95%) d'acide phényl-4 oxo-2 oxa-1 spiro (4,4)nonane carboxylique-3 présentant un point de fusion F=148—150°C (décomposition).

21g de l'acide obtenu comme ci-dessus sont traités sous refroidissement par 56ml de solution alcoolique de diméthylamine à 33%, puis on ajoute goutte à goutte, sous agitation et refroidissement, 13ml de formol à 35%. Le mélange obtenu est agité à température ambiante pendant 48h puis porté à ébullition pendant 1h. Après évaporation des solvants sous pression réduite, on obtient un résidu auquel on ajoute de l'eau. Le mélange est extrait à l'éther, puis les couches éthérées sont lavées à l'eau, par une solution de carbonate de sodium à 5%, et séchées sur sulfate de sodium. Après recristallisation dans un mélange d'éther et de n-pentane on obtient 17,7g de (diméthylaminométhyl)-3 phényl-4 oxa-1 spiro (4,4)nonane-one-2, présentant un point de fusion F=76—77°C (rendement 81%).

Analyse $C_{17}H_{23}NO_2$ (273,362)

| | | | |
|---|---|---|---|
| % calculé | C:74,69 | H:8,48 | N:5,12 |
| % trouvé | C:74,44 | H:8,30 | N:4,93 |

La structure du produit est confirmée par les spectres IR et RMN.

## Exemple 2

### (Diméthylaminométhyl)-3 phényl-4 oxa-1 spiro (4,5) décane-one-2

On procède comme indiqué dans l'exemple 1 en remplaçant l'acide $\beta$-cyclopentyl-$\beta$-hydroxy-hydro-cinnamique par de l'acide $\beta$-cyclohexyl-$\beta$-hydroxy-hydrocinnamique. Pendant l'hydrolyse du complexe il se forme un produit visqueux qui cristallise par refroidissement et frottement. Après filtration, lavage à l'eau et au n-pentane, séchage sur $P_2O_5$ et recristallisation dans un mélange d'éther et de n-pentane, on obtient la phényl-4 oxa-1 spiro(4,5)décane-one-2 avec un rendement de 99%, dont le point de fusion est F=104—105°C.

La spirolactone ainsi obtenue est traitée comme indiqué dans l'exemple 1, par une solution de carbonate de méthyle et de magnésium dans le diméthylformamide, en portant le mélange à ébullition. On obtient ainsi avec un rendement de 86% l'acide phényl-4 oxo-2 oxa-1 spiro(4,5) décane carboxylique-3 présentant un point de fusion F=158—160°C (décomposition).

L'acide ainsi obtenu est traité comme indiqué dans l'exemple 1 par une solution alcoolique de diméthylamine, et on obtient avec un rendement de 91% la (diméthylaminométhyl)-3 phényl-4 oxa-1 spiro(4,5)décane-one-2 présentant un point de fusion F=117—119°C.

Analyse $C_{18}H_{25}NO_2$ (287,368)

| | | | |
|---|---|---|---|
| % calculé | C:75,22 | H:8,77 | N:4,87 |
| % trouvé | C:75,48 | H:8,67 | N:5,04 |

On prépare le chlorhydrate par les méthodes usuelles, en ajoutant une solution éthanolique d'acide chlorhydrique dans une solution éthérée de la base. Le chlorhydrate présente un point de fusion F=206—207°C.

Analyse $C_{18}H_{26}ClNO_2$ (323,853)

| | | | | |
|---|---|---|---|---|
| % calculé | C:66,75 | H:8,09 | Cl:10,95 | N:4,33 |
| % trouvé | C:66,36 | H:7,83 | Cl:11,30 | N:4,23 |

## Exemple 3

### (Diméthylaminométhyl)-3 (p-tolyl)-4 oxa-1 spiro(4,5)décane-one-2

En procédant comme indiqué dans l'exemple 1, mais en remplaçant l'acide $\beta$-cyclopentyl-$\beta$-

hydroxy-hydrocinnamique par l'acide p-méthyl-β-cyclohexyl-β-hydroxy-hydrocinnamique on obtient, avec un rendement de 92%, la (p-tolyl)-4 oxa-1 spiro(4,5)décane-one-2 présentant un point de fusion F=92—93°C.

La spirolactone ainsi obenue est traitée comme indiqué dans l'exemple 1, par une solution de carbonate de méthyle et de magnésium dans le diméthylformamide, en portant le mélange à ébullition. On obtient ainsi, avec un rendement de 79% l'acide (p-tolyl)-4 oxo-2 oxa-1 spiro(4,5)décane carboxylique-3 présentant un point de fusion F=171—172°C (décomposition).

L'acide obtenu comme indiqué ci-dessus est traité sous refroidissement par une solution alcoolique de diméthylamine comme indiqué dans l'exemple 1. On obtient ainsi, avec un rendement de 60% la (diméthylaminométhyl)-3 (p-tolyl)-4 oxa-1 spiro(4,5)décane-one-2, présentant un point de fusion F=133—134°C.

Analyse $C_{19}H_{27}NO_2$ (301,414)

| | | | |
|---|---|---|---|
| % calculé | C:75,71 | H:9,03 | N:4,65 |
| % trouvé | C:75,76 | H:9,07 | N:4,65 |

Le chlorhydrate du composé ci-dessus présente un point de fusion F=221—222°C.

Analyse $C_{19}H_{28}ClNO_2$ (337,879)

| | | | | |
|---|---|---|---|---|
| % calculé | C:67,54 | H:8,35 | Cl:10,19 | N:4,15 |
| % trouvé | C:67,28 | H:8,11 | Cl:10,29 | N:4,44 |

Exemple 4

Iodométhylate de la (diméthylaminométhyl)-3 phényl-4 oxa-1 spiro(4,4)nonane-one-2

On agite pendant 72 h à température ambiante 5,5g de (diméthylaminométhyl)-3 phényl-4 oxa-1 spiro(4,4) nonane-one-2 obtenue comme indiqué dans l'exemple 1, en présence de 6ml de iodure de méthyle dans 60ml d'acétone anhydre. On ajoute encore 2ml de iodure de méthyle, et on porte le mélange à ébullition pendant 3 h. Après refroidissement à température ambiante, on ajoute de l'éther anhydre. Le sel formé est filtré et recristallisé dans un mélange d'éthanol et d'éther anhydre.

On obtient ainsi 7,7g (rendement 93%) de iodométhylate présentant un point de fusion F=185—186°C.

Analyse $C_{18}H_{26}INO_2$ (415,306)

| | | | | |
|---|---|---|---|---|
| % calculé | C:52,05 | H:6,31 | I:30,56 | N:3,37 |
| % trouvé | C:51,67 | H:6,37 | I:30,62 | N:3,55 |

Exemple 5

Iodométhylate de la (diméthylaminométhyl)-3 phényl-4 oxa-1 spiro (4,5) décane-one-2.

On procède comme indiqué dans l'exemple 4, à partir de la spirolactone obtenue comme indiqué dans l'exemple 2. On obtient ainsi, avec un rendement de 94%, le iodométhylate présentant un point de fusion F=218—220°C.

Analyse $C_{19}H_{28}INO_2$ (429,332)

| | | | | |
|---|---|---|---|---|
| % calculé | C:53,15 | H:6,57 | I:29,56 | N:3,26 |
| % trové | C:53,18 | H:6,53 | I:29,50 | N:3,42 |

Exemple 6

(diméthylaminométhyl)-3 phényl-4 oxa-1 spiro(4,5)décane.

17g de(diméthylaminométhyl)-3 phényl-4 oxa-1 spiro (4,5)décane-one-2, en solution dans 150ml d'éther anhydre, sont ajoutés lentement et sous agitation dans une suspension de 5g d'hydrure de lithium-aluminium dans 300ml de tétrahydrofuranne anhydre, puis le mélange est porté à ébullition pendant 7h. Le complexe qui se forme est hydrolysé à froid et sous agitation, en ajoutant goutte à goutte de l'eau et une solution de soude à 10%. Après filtration des hydroxydes d'aluminium et de lithium, les solvants sont évaporés sous pression réduite, et le résidu, solidifié par refroidissement, est recristallisé dans un mélange d'éther et de n-pentane.

On obtient ainsi, avec un rendement de 87%, le (phényl-1 diméthylaminométhyl-2 hydroxy-3) propyl-1 cyclohexanol présentant un point de fusion F=146—147°C.

L'amino-diol obtenu comme indiqué ci-dessus est mis en solution dans 300ml de pyridine anhydre et on ajoute sous agitation et refroidissement 10,4g de chlorure de p-bromobenzène sulfonyle par petites quantités successives. Le mélange est agité pendant 20 h à température ambiante, puis versé dans de l'eau. On extrait au chloroforme, et les couches chloroformiques sont lavées à l'eau puis séchées sur sulfate de sodium. Le solvant est éliminé sous vide, et le résidu est soumis à une chromatographie sur colonne d'alumine neutre (1g de résidu pour 30g d'alumine), en utilisant l'éther comme éluant. Après évaporation des fractions correspondantes et purification par recristallisation dans un mélange d'éther et de n-pentane on obtient, avec un rendement de 78%, le (diméthylaminométhyl)-3 phényl-4 oxa-1 spiro(4,5)décane présentant un point de fusion F=65°C.

Analyse $C_{18}H_{27}NO$ (273,404)

| | | | |
|---|---|---|---|
| % calculé | C:79,07 | H:9,95 | N:5,12 |
| % trouvé | C:79,14 | H:10,14 | N:5,01 |

On prépare de manière usuelle le chlorhydrate correspondant présentant un point de fusion 201—202°C.

Analyse $C_{18}H_{28}ClNO$ (309,869)

| | | | | |
|---|---|---|---|---|
| % calculé | C:69,76 | H:9,11 | Cl:11,44 | N:4,52 |
| % trouvé | C:69,79 | H:8,79 | Cl:11,36 | N:4,47 |

Exemple 7

(diméthylaminométhyl)-3 p-chlorophényl-4 oxa-1 spiro(4,4)nonane-one-2.

Ce produit est obtenu par le même procédé que dans l'exemple 1, en prenant comme composé de départ la cyclopentyl-p-chlorphényl-cétone (décrite dans le brevet belge 634.208) à la place de la cyclopentylphénylcétone. On forme ainsi l'acide $\beta$-cyclopentyl $\beta$-hydroxy chloro-4' hydrocinnamique que l'on transforme en p-chloro-phényl-4 oxa-1 spiro (4,4) nonane-one-2.

Après formation de l'acide correspondant (F=153—155°C) comme indiqué dans l'exemple 1, on fait agir la diméthylamine en solution dans l'alcool, en présence de formol, pour obtenir le composé recherché.

Point de fusion: F=92—94°C (éther-n-pentane).

Exemple 8

Chlorhydrate de (diméthylaminométhyl)-3 p-chlorophényl-4 oxa-1 spiro (4,4) nonane.

On procède comme indiqué dans l'exemple 6, en remplaçant la (diméthylaminométhyl)-3 phényl-4 oxa-1 spiro (4,5) décane-one-2 par la (diméthylaminométhyl)-3 p-chlorophényl-4 oxa-1 spiro (4,4) nonane-one-2, que l'on ajoute lentement à une suspension d'hydrure de lithium-aluminium dans le tétrahydrofuranne anhydre.

L'amino-diol obtenu est transformé en (diméthylaminométhyl)-3 p-chlorophényl-4 oxa-1 spiro (4,4) nonane-one-2 par action de chlorure de p-bromobenzène sulfonyle dans la pyridine.

On fait ensuite agir l'acide chlorhydrique pour obtenir le chlorhydrate recherché, en solution dans un mélange éthanol-éther.

Point de fusion: F=221—223°C

Exemple 9

Chlorhydrate de N-pipéridinométhyl-3 p-chlorophényl-4 oxa-1 spiro (4,5)décane-one-2

On procède comme indiqué dans l'exemple 1 en prenant comme composé de départ la cyclohexyl-p-chlorophényl cétone (décrite dans le brevet US 3308.159) à la place de la cyclopentylphénylcétone. On forme ainsi l'acide $\beta$-cyclo-hexyl $\beta$-hydroxy chloro-4'-hydrocinnamique que l'on transforme en p-chlorophényl-4 oxa-1 spiro (4,5) décane-one-2.

Après formation de l'acide correspondant (F=156—159°C décomp.) suivant la technique décrite dans l'exemple 1, on fait agir la pipédirine en solution dans l'alcool pour obtenir la N-pipéridinométhyl-3 p-chloro-phényl-4 oxa-1 spiro (4,5) décane-one-2 que l'on transforme en son chlorhydrate par action de l'acide chlorhydrique, dans un mélange d'éthanol-éther diéthylique.

Point de fusion: F=214—216°C (décomp.)

Exemple 10

Chlorhydrate de N-pipéridinométhyl-3 p-chloro-phényl-4 oxa-1 spiro (4,5) décane

On procède comme indiqué dans l'exemple 6, en utilisant la N-pipéridinométhyl-3 p-chlorophényl-4 oxa-1 spiro (4,5) décane-one-2 que l'on ajoute lentement à une suspension d'hydrure de lithium-aluminium dans le tétrahydrofuranne anhydre.

L'amino-diol obtenu est transformé en N-pipéridinométhyl-3 p-chlorphényl-4 oxa-1 spiro (4,5) décane par action du chlorure de p-bromobenzène sulfonyle dans la pyridine.

On obtient le chlorhydrate dans un mélange éthanoléther diéthylique, suivant la technique usuelle, par action de l'acide chlorhydrique.

Point de fusion: F=281—283°C

Exemple 11

Chlorhydrate de N-pipéridinométhyl-3 p-tolyl-4 oxa-1 spiro (4,5) décane-one-2.

On obtient ce produit en procèdant comme indiqué dans l'exemple 9, mais en prenant comme composé de départ la cyclohexyl p-tolyl cétone. La N-pipéridinométhyl-3 p-tolyl-4 oxa-1 spiro (4,5) décane-one-2 est transformée en son chlorhydrate par action de l'acide chlorhydrique dans un mélange éthanol-éther diéthylique.

Point de fusion: F=214—216°C (décomp.)

Exemple 12

Chlorhydrate de N-pipéridinométhyl-3 p-tolyl-4 oxa-1 spiro (4,5) décane.

On procède comme indiqué dans l'exemple 6 en utilisant la N-pipéridinométhyl-3 p-tolyl-4 oxa-1 spiro(4,5) décane-one-2 que l'on ajoute lentement à une suspension d'hydrure de lithium-aluminium dans le tétrahydrofuranne anhydre pour former l'amino-diol correspondant.

Le N-pipéridinométhyl-3 p-tolyl-4 oxa-1 spiro(4,5) décane est obtenu à partir de l'amino-diol par action du chlorure de p-bromobenzène sulfonyle dans la pyridine.

On prépare ensuite le chlorhydrate de manière usuelle en faisant agir l'acide chlorhydrique, et l'on extrait par un solvant constitué par un mélange éthanol-éther diéthylique.

Point de fusion: F=283—286°C (décomp.)

**Revendications**

1. Dérivés spiranniques de formule générale (I):

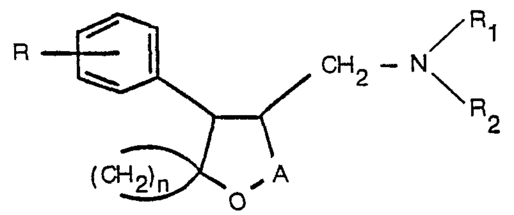

dans laquelle R est un atome d'hydrogène ou d'halogène, ou un groupe méthyle; $R_1$ et $R_2$, identiques ou différents, représentent un atome d'hydrogène ou un groupe méthyle ou représentent ensemble une chaîne carbonée formant avec l'atome d'azote auquel ils sont rattachés, un hétérocycle à 5 ou 6 chaînons; A représente un groupe

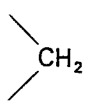 ou 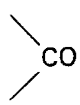

et n est 4 ou 5, ainsi que leurs sels d'addition d'acides.

2. Dérivés sprianniques selon la revendication 1, caractérisés en ce que A représente un groupe

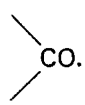

3. Dérivés spiranniques selon la revendication 1, caractérisés en ce que A est un groupe

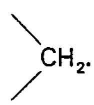

4. Dérivés spiranniques selon la revendication 2, caractérisés en ce qu'ils sont choisis parmi la (diméthylaminométhyl)-3 phényl-4 oxa-1 spiro (4,4) nonane-one-2, la (diméthylaminométhyl)-3 phényl-4 oxa-1 spiro (4,5) décane-one-2, la (diméthylaminométhyl)-3 (p-tolyl)-4 oxa-1 spiro (4,5) décane-one-2, et la (diméthylaminométhyl)-3 p-chloro-phényl-4 oxa-1 spiro (4,4) nonane-one-2.

5. Dérivés spiranniques selon la revendication 2, caractérisés en ce qu'ils sont choisis parmi la N-pipéridinométhyl-3 p-chlorophényl-4 oxa-1 spiro (4,5) décane-one-2 et la N-pipéridinométhyl-3 p-tolyl-4 oxa-1 spiro (4,5) décane-one-2.

6. Dérivés spiranniques selon la revendication 3, caractérisés en ce qu'ils sont choisis parmi le (diméthylaminométhyl)-3 phényl-4 oxa-1 spiro (4,5) décane, et le (diméthylaminométhyl)-3 p-chloro-phényl-4 oxa-1 spiro (4,4) nonane.

7. Dérivés spiranniques selon la revendication 3, caractérisés en ce qu'ils sont choisis parmi le N-pipéridinométhyl-3 p-chlorophényl-4 oxa-1 spiro (4,5) décane et le N-pipéridinométhyl-3 p-tolyl-4 oxa-1 spiro (4,5) décane.

8. Procédé de préparation de dérivés spiranniques selon l'une quelconque des revendications précédentes, caractérisé en ce que l'on traite un $\beta$-hydroxyacide de formule générale (II):

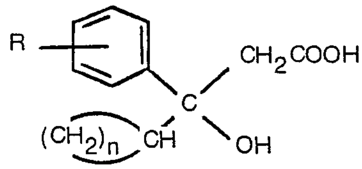

dans laquelle R et n ont la même signification que dans la formule (I) de la revendication 1, par un acide fort, suivi d'une hydrolyse pour former une $\gamma$-spirolactone de formule générale (III):

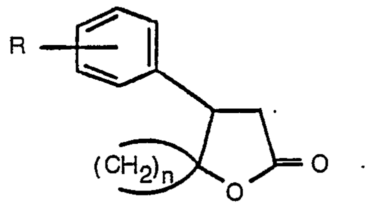

dans laquelle R et n conservent la définition indiquée dans la formule générale I, de la revendication 1, que l'on traite par le carbonate de méthyle et de magnésium, pour former l'$\alpha$-carboxy-$\gamma$-spirolactone correspondante sur laquelle on fait réagir une amine aliphatique appropriée, en présence de formol ou de paraformaldéhyle suivant la réaction de Mannich., pour former le composé de formule générale (I) de la revendication 1, où A est —CO—, et, le cas échéant, on effectue une ouverture réductive du cycle lactonique par l'hydrure de lithium-aluminium, puis une cyclisation par déshydratation interne, par action du chlorure de p-bromo-benzène sulfonyle dans la pyridine, pour former le composé de formule générale (I) de la revendication 1, où A est —$CH_2$—.

9. Procédé selon la revendication 8, caractérisé en ce que l'acide fort est l'acide sulfurique concentré, en présence d'un oléum.

10. Médicament, caractérisé en ce qu'il est constitué par un dérivé spirannique selon l'une quelconque des revendications 1 à 7, ou un de ses sels pharmaceutiquement acceptables.

11. Composition pharmaceutique caractérisée en ce qu'elle contient, à titre de principe actif, un médicament selon la revendication 10, associé à un ou plusieurs excipients pharmaceutiquement acceptables.

## Claims

1. Spiro derivatives of the general formula (I):

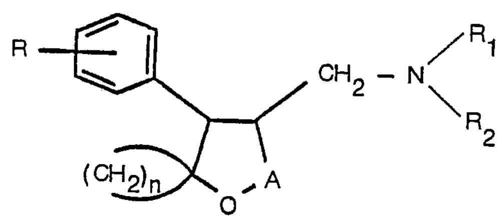

wherein R is a hydrogen atom, a halogen atom, or a methyl group; $R_1$ and $R_2$, which may be the same or different, represent a hydrogen atom or a methyl group or together represent carbon atoms forming a 5- or 6-membered heterocyclic ring with the nitrogen atom to which they are linked; A represents a

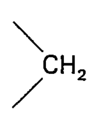 group or a 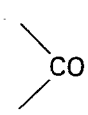

group; and n is 4 or 5 and the acid addition salts thereof.

2. Spiro derivatives according to Claim 1, characterized in that A represents a

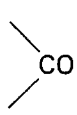

group.

3. Spiro derivatives according to Claim 1, characterized in that A is a

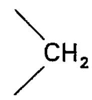

group.

4. Spiro derivatives according to Claim 2, characterized in that they are selected from the group consisting of (dimethylaminomethyl)-3 phenyl-4 oxa-1 spiro(4,4)nonane-one-2, (dimethylaminomethyl)-3 phenyl-4 oxa-1 spiro(4,5)decane-one-2, (dimethylaminomethyl-3 (p-tolyl)-4 oxa-1 spiro(4,5)decane-one-2, and (dimethylaminomethyl)-3 p-chlorophenyl-4 oxa-1 spiro(4,4)nonane-one-2.

5. Spiro derivatives according to Claim 2, characterized in that they are selected from the group consisting of N-piperidinomethyl-3 p-chlorophenyl-4 oxa-1 spiro(4,5)-decane-one-2, and N-piperidinomethyl-3 p-tolyl-4 oxa-1 spiro(4,5)decane-one-2.

6. Spiro derivatives according to Claim 3, characterized in that they are selected from the group consisting of (dimethylaminomethyl)-3 phenyl-4 oxa-1 spiro(4,5)decane, and (dimethylaminomethyl)-3 p-chlorophenyl-4 oxa-1 spiro(4,4)nonane.

7. Spiro derivatives according to Claim 3, characterized in that they are selected from the group consisting of N-piperidinomethyl-3 p-chlorophenyl-4 oxa-1 spiro(4,5)decane and N-piperidinomethyl-3 p-tolyl-4 oxa-1 spiro(4,5)decane.

8. Process for preparation of the spiro derivatives according to any of the preceding claims, characterized in that it comprises treating $\beta$-hydroxyacid of the general formula (II)

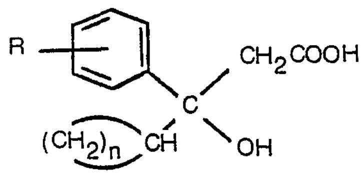

wherein R and n have the same significance as in the general formula (I) of Claim 1 with a strong acid, then hydrolyzing the product obtained to form a $\gamma$-spirolactone of the general formula (III):

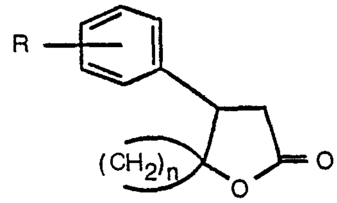

wherein R and n have the same significance as in the general formula (I) of claim 1, treating the product thus obtained with methyl and magnesium carbonate to form the corresponding $\alpha$-carboxy-$\gamma$spirolactone, which is caused to react with an aliphatic amine in the presence of formaldehyde or paraformaldehyde in a Mannich reaction, to form the compound of the general formula (I) of claim 1 wherein A is —CO—, and reductively opening, if necessary, the lactone ring using lithium aluminium hydride, and cyclizing by internal dehydration, using p-bromobenzene sulfonyl chloride in pyridine, to form the compound of the general formula (I) of claim 1 wherein A is —$CH_2$—.

9. Process according to Claim 6, characterized in that the strong acid is concentrated sulfuric acid in the presence of oleum.

10. Medicine, characterized in that it comprises a spiro derivative according to any of claims 1 to 7, or a pharmaceutically acceptable salt thereof.

11. A pharmaceutical composition characterized in that it comprises a medicine according to Claim 10, as an active ingredient, along with one or several pharmaceutically acceptable diluents.

**Patentansprüche**

1. Spiro-Derivate der allgemeinen Formel (I):

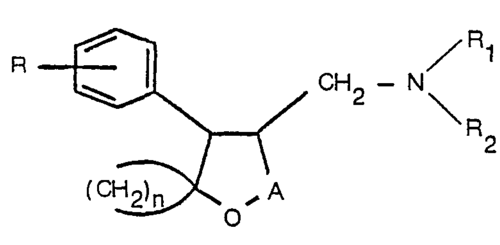

worin R ein Wasserstoff- oder Halogenatom oder eine Methylgruppe ist; $R_1$ und $R_2$, gelich oder verschieden, ein Wasserstoffatom oder eine Methylgruppe oder zusammen eine Kohlenstoffkette, wobei mit dem Stickstoffatom, mit welchem sie verbunden sind, eine aus 5 oder 6 Gliedern bestehende heterocyklysche Gruppe gebildet wird, bedeuten; A eine

$>CH_2$-oder eine $>CO$-Gruppe

bedeutet und n ist 4 oder 5, sowie ihre Zusatzsalze der Saüren.

2. Spiro-Derivate gemäss Anspruch 1, dadurch gekennzeichnet, dass A eine

$>CO$-Gruppe

ist.

3. Spiro-Derivate gemäss Anspruch 1, dadurch gekennzeichnet, dass A eine

$>CH_2$-Gruppe

ist.

4. Spiro-Derivate gemäss Anspruch 2, dadurch gekennzeichnet, dass sie aus der 3-(Dimethylaminomethyl)-4-Phenyl-1-Oxa-(4,4)-Spiro-2-Nonanone, der 3-(Dimethylaminomethyl)-4-Phenyl-1-Oxa-(4,5)-Spiro-2-Dekanone, der 3-(Dimethylaminomethyl)-4-p-tolyl-1-oxa-(4,5)-Spiro-2-Dekanone und der 3-(Dimethylaminomethyl)-4-p-chlorphenyl-1-Oxa-(4,4)-Spiro-2-Nonanone, ausgewählt sind.

5. Spiro-Derivate gemäss Anspruch 2, dadurch gekennzeichnet, dass sie aus der 3-N-Piperidinmethyl-4-p-chlorphenyl-1-Oxa-(4,5)-Spiro-2-Dekanone und der 3-N-Piperidinmethyl-4-p-tolyl-1-Oxa-(4,5)-Spiro-2-Dekanone ausgewählt sind.

6. Spiro-Derivate gemäss Anspruch 3, dadurch gekennzeichnet, dass sie aus dem 3-(Dimethylaminomethyl)-4-Phenyl-1-Oxa-(4,5)-Spiro-Dekan und dem 3-(Dimethylaminomethyl)-4-p-chlorphenyl-1-Oxa-(4,4)-Spiro-Nonan ausgewählt sind.

7. Spiro-Derivate gemäss Anspruch 3, dadurch gekennzeichnet, dass sie aus dem 3-N-Piperidinmethyl-4-p-chlorphenyl-1-Oxa-(4,5)-Spiro-Dekan und dem 3-N-Piperidinmethyl-4-p-tolyl-1-Oxa-(4,5)-Spiro-Dekan ausgewählt sind.

8. Verfahren zur Herstellung der Spiro-Derivate gemäss eines beliebigen der vorstehenden Ansprüchen, dadurch gekennzeichnet, dass man eine $\beta$-Hydroxysäure der allgemeinen Formel (II):

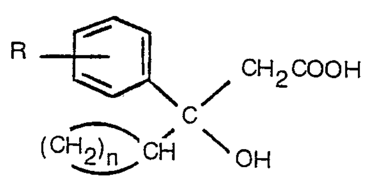

worin R und n die gleiche Bedeutung wie in der Formel (I) des Anspruches 1 haben, mit einer starken Säure behandelt, danach zur Bildung des $\gamma$-Spirolactons der allgemeinen Formel (III):

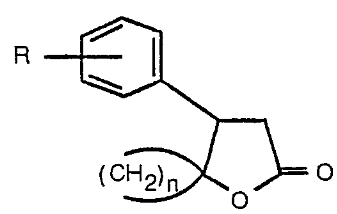

in welcher R und n die in der allgemeinen Formel I des Anspruches 1 angegebene Bedeutung beibehalten, hydrolysiert, welches mit dem Methylund Magnesiumkarbonat zur Bildung des entsprechenden $\alpha$-Carboxy- $\gamma$-Spirolactons behandelt wird, auf welchem man in Gegenwart Formol oder Paraformaldehyd entsprechend der Mannich. Reaktion ein geeignetes aliphatisches Amin reagieren lässt, um die Verbindung der allgemeinen Formel (I) des Anspruches 1, wobei A eine —CO-Gruppe ist, zu gewinnen und gegebenfalls den Lactonring durch das Lithiumaluminiumhydrid reduktiv unterbricht, um sodann durch innere Deshydratisierung durch Einwirkung des p-Bromobenzolsulfonylchlorids im Pyridin

zu zyklisieren, um die Verbindung der allgemeinen Formel (I) des Anspruches 1, wobei A $—CH_2—$ ist, zu gewinnen.

9. Verfahren gemäss Anspruch 8, dadurch gekennzeichnet, dass die Saüre die konzentrierte Schwefelsäure in Gegenwart eines Oleums ist.

10. Arzneimittel, dadurch gekennzeichnet, dass es sich aus einem Spiro-Derivat gemäss eines beliebigen Anspruches 1 bis 7 oder aus einem seiner pharmazeutisch zulässigen Salze susammensetzt.

11. Pharmazeutische Zusammensetzung, dadurch gekennzeichnet, dass sie als Wirkstoff ein Arzneimittel gemäss Patentanspruch 10 zusammen mit einem oder mehreren pharmazeutisch zulässigen Bindemitteln enthält.